(19) Europäisches Patentamt
European Patent Office
Office européen des brevets

(11) **EP 4 675 465 A1**

(12) **EUROPÄISCHE PATENTANMELDUNG**

(43) Veröffentlichungstag:
    **07.01.2026  Patentblatt 2026/02**

(21) Anmeldenummer: **25186551.5**

(22) Anmeldetag: **01.07.2025**

(51) Internationale Patentklassifikation (IPC):
    **G06F 18/28** (2023.01)     **G06F 21/62** (2013.01)
    **G06F 17/18** (2006.01)

(52) Gemeinsame Patentklassifikation (CPC):
    **G06F 18/28; G06F 17/18; G06F 21/6254;**
    G16H 50/70

(84) Benannte Vertragsstaaten:
    **AL AT BE BG CH CY CZ DE DK EE ES FI FR GB GR HR HU IE IS IT LI LT LU LV MC ME MK MT NL NO PL PT RO RS SE SI SK SM TR**
    Benannte Erstreckungsstaaten:
    **BA**
    Benannte Validierungsstaaten:
    **GE KH LA MA MD TN**

(30) Priorität:  **02.07.2024  DE 102024002388**

(71) Anmelder: **Bundesdruckerei GmbH**
    **10969 Berlin (DE)**

(72) Erfinder:
    • **Graupner, Hendrik**
      **10969 Berlin (DE)**
    • **Menter, Josef**
      **10969 Berlin (DE)**
    • **Kauer-Bonin, Josef**
      **10969 Berlin (DE)**
    • **Dewes, Andreas**
      **10625 Berlin (DE)**

(54) **VERFAHREN, DATENVERARBEITUNGSVORRICHTUNG UND SYSTEM ZUR BEURTEILUNG UND VERÄNDERUNG VON SYNTHESEDATEN**

(57)    Die Erfindung betrifft ein computerimplementiertes Verfahren und eine Datenverarbeitungsvorrichtung zur selektiven Veränderung eines Synthesedatensatzes. Das Verfahren umfasst das Erhalten eines Originaldatensatzes, umfassend eine Vielzahl von Originaldatenpunkten, und eines, basierend auf dem Originaldatensatz synthetisierten, Synthesedatensatzes, umfassend eine Vielzahl von Synthesedatenpunkten. Das Verfahren umfasst weiterhin ein Bestimmen einer Menge kritischer Synthesedatenpunkte aus der Vielzahl der Synthesedatenpunkte durch eine gemeinsame Auswertung einer ersten Evaluierungsfunktion und einer zweiten Evaluierungsfunktion des Originaldatensatzes und Synthesedatensatzes, und ein Verändern mindestens eines der kritischen Synthesedatenpunkte.

Figur 1

**Beschreibung**

Die vorliegende Erfindung betrifft ein computerimplementiertes Verfahren zur automatisierten Bewertung und selektiven Veränderung eines Synthesedatensatzes, sowie eine entsprechende Datenverarbeitungsvorrichtung und ein entsprechendes System.

Unter Datensynthese wird die Generierung künstlicher Daten verstanden, die mit dem Ziel generiert werden, Eigenschaften von Originaldaten, die durch Messung oder Abfrage von Ereignissen gewonnen wurden, abzubilden. Die Generierung synthetischer Daten dient beispielsweise dazu, sensible Daten zu verschleiern, aber gleichzeitig statistische Eigenschaften der Originaldaten zu erhalten. Die schützenswerten Daten können hierbei insbesondere sicherheitsrelevante, sowie persönliche oder personenbezogene Daten sein, die aus Gründen des Datenschutzes nicht weitergegeben und zu statistischen Zwecken verarbeitet werden dürfen.

Synthetische Daten, die die Eigenschaften der Originaldaten möglichst gut wiedergeben sind in der Infrastrukturplanung von großer Bedeutung. Beispielsweise können medizinische Synthesedaten, d.h. synthetische Daten, die einen Datensatz mit medizinischen Messdaten und/oder Patientendaten widerspiegeln, zur Planung von medizinischen Kapazitäten verwendet werden. Synthetische Daten, die basierend auf Verkehrsdaten generiert wurden können zur Planung von Verkehrsinfrastruktur und zur Simulation von Verkehrsflüssen verwendet werden.

Verfahren zur Generierung synthetischer, anonymer Daten aus einem Originaldatensatz, sogenannte Datensyntheseverfahren, müssen einerseits sicherstellen, dass die statistischen Eigenschaften des Originaldatensatzes im Synthesedatensatz repräsentiert bleiben. Gleichzeitig muss sichergestellt werden, dass aus dem Synthesedatensatz keine Rückschlüsse auf einzelnen Originaldatenpunkte möglich sind.

Ein anonymer Datensatz darf gemäß Datenschutzbestimmungen, im Gegensatz zu dem Originaldatensatz, außerhalb der datenerhebenden Organisation geteilt werden und darf außerhalb einer der datenerhebenden Organisation zugeordneten sicheren Umgebung übertragen und verarbeitet werden. Der anonyme Datensatz kann hierbei entstanden sein durch sogenannte Datensyntheseverfahren oder alternativ auch durch Anonymisierung bzw. Verrauschen der Originaldaten, durch gezielte Veränderung einzelner Datenpunkte etc.

Mögliche Ansätze zur Datensynthese bestehen aus Verfahren basierend auf künstlicher Intelligenz oder mit weiteren Verfahren des maschinellen Lernens oder durch gezielte oder randomisierte Veränderungen der Originaldaten.

Verfahren zur Beurteilung der Güte eines synthetischen Datensatzes beziehen sich üblicherweise auf den Vergleich statistischer Eigenschaften der Originaldaten mit statistischen Eigenschaften des synthetischen Datensatzes. Ziel dieses Vergleiches ist es, die Signifikanz der synthetischen Daten zu beurteilen und zu garantieren, damit die synthetischen Daten dann anstelle der Originaldaten zu statistischen Zwecken verwendet werden können. Diese Art des "Gütesiegels" trifft jedoch keine Aussage über den Grad der Privatheit oder möglicher Sicherheitsverletzungen der synthetischen Daten.

Datenerzeugungssysteme basierend auf maschinellem Lernen (auch "ML-Systeme" genannt) oder generativer künstlicher Intelligenz sind zwar potenziell in der Lage, große Mengen von Synthesedaten in geringer Zeit zu erzeugen, bei Systemen des maschinellen Lernens besteht aber aufgrund des Blackbox-Charakters der verwendeten Datenerzeugung das Risiko, dass einzelne Originaldatenpunkte nicht ausreichend verschleiert werden.

Es gibt zwar Ansätze, die Parameter des ML-Systems so zu wählen, dass die Wahrscheinlichkeit für eine Reproduktion einzelner Originaldatenpunkte im Synthesedatensatz reduziert wird, diese Ansätze betreffen aber nur die Parameter des ML-Systems, beurteilen aber nicht die Privatheit oder Sicherheit des generierten Datensatz und können daher, insbesondere bei sensiblen Originaldaten, eine händische Beurteilung und Freigabe des Synthesedatensatzes bisher nicht ersetzen.

Da die Privatsphäreverletzung oder ein Sicherheitsmangel eines Synthesedatensatzes bereits daraus resultieren kann, dass ein einzelner Synthesedatenpunkt sensible Daten des Originaldatensatzes enthält, sind statistische Tests insbesondere bei großen Datensätzen nicht ausreichend, um privatsphäreverletzende oder sicherheitskritische Synthesedatenpunkte zu identifizieren. Synthetische Datensätze sind aber nur dann sinnvoll nutzbar, wenn sie keine sicherheitskritischen und privatsphärenverletzenden Daten enthalten, da sie nur dann außerhalb einer sicheren Umgebung für technische Planungen und Simulationen, beispielsweise in der Infrastruktur nutzbar sind.

Im Fokus der vorliegenden Anmeldung liegen somit Verfahren zur Verbesserung der Sicherheit und des Privatsphäregrades eines anonymisierten/künstlichen Datensatzes in Bezug auf einen Originaldatensatz um somit die synthetischen Datensätze zur öffentlichen Nutzung beispielsweise in der Infrastrukturplanung verwendbar zu machen.

Der Erfindung liegt daher die Aufgabe zugrunde, die Privatheit und somit die Datensicherheit eines Synthesedatensatzes zu verbessern.

Die Aufgabe wird durch die Verfahren und Gegenstände der unabhängigen Patentansprüche gelöst. Bevorzugte Ausführungsformen der Erfindung ergeben sich aus den in den Unteransprüchen genannten Merkmalen und weiterhin auch aus der vorliegenden Offenbarung als Ganzes.

Ein erster Aspekt der Erfindung betrifft ein computerimplementiertes Verfahren zur selektiven Veränderung eines Synthesedatensatzes. Das Verfahren beinhaltet, als erste Schritte, das Erhalten eines Originaldatensatzes, umfassend eine Vielzahl von Originaldatenpunkten, wobei jeder der Originaldatenpunkte Originalwerte für eine Vielzahl von Attributen umfasst und das Erhalten eines basierend auf dem Originaldatensatzes generierten Synthesedatensatzes, umfassend eine Vielzahl von Synthesedatenpunkten, wobei jeder der Synthesedatenpunkte Synthesewerte für eine Vielzahl der Attribute umfasst.

Ein Datensatz (englisch "data set") im Sinn der vorliegenden Anmeldung bezeichnet hierbei eine strukturierte Sammlung von Datenpunkten, die sich üblicherweise auf ein bestimmtes Thema beziehen und/oder für einen bestimmten Zweck erhoben bzw. gemessen wurden. Ein Datensatz bezieht sich auf eine vorgegebene Menge von Attributen, wobei die einzelnen Datenpunkte jeweils Werte für zumindest eine Teilmenge der Attribute des Datensatzes beinhalten. Die einzelnen Werte können hierbei Messwerte, Zahlen, Textdaten oder andere Werte sein, die den jeweiligen Datenpunkt beschreiben.

Ein Datensatz kann beispielsweise in Tabellenform dargestellt werden, wobei jede Zeile einen Datenpunkt spezifiziert und die Spalten die jeweiligen Attribute abbilden. Alternativ kann die Tabelle auch transponiert (mit den Datenpunkten in Spaltenform) gespeichert werden oder der Datensatz kann auf andere Art und Weise, wie im Stand der Technik bekannt, abgespeichert oder abgebildet werden. Somit kann ein Datensatz im mathematischen Sinne als Matrix und jeder einzelne Datenpunkt als Vektor betrachtet werden.

Ein Datensatz definiert im mathematisch-statistischen Sinn eine mehrdimensionale Zufallsverteilung über die Attribute des Datensatzes.

Somit können für einen Datensatz auch Randverteilungen bezüglich einer Teilmenge von Attributen des Datensatzes betrachtet werden, die jeweils die absoluten oder relativen Häufigkeiten bezüglich Kombinationen der jeweiligen Teilmenge der Attribute beschreiben.

Die Werte der verschiedenen Attribute eines Datensatzes können statistisch unabhängig voneinander sein oder sie können korreliert sein, so dass bestimmte Werte eines ersten Attributes mit höherer Wahrscheinlichkeit zusammen mit (anderen) bestimmten Werten eines zweiten Attributs auftreten.

Jedem Attribut eines Datensatzes kann üblicherweise ein Werteraum zugeordnet werden. Beispiele für Werteräume von Attributen können numerische Werte (auch als stetige Werte bezeichnet) oder kategorielle Werte (beispielsweise in Form einer Auswahl aus einer Liste) sein.

Der Originaldatensatz im Sinne der vorliegenden Anmeldung umfasst vorzugsweise eine große Anzahl, beispielsweise mehr als 1.000, mehr als 10.000 oder mehr als 100.000 Datenpunkte und jeder der Datenpunkte beinhaltet Originalwerte bezüglich einer Vielzahl von Attributen. Vorzugsweise sind mindestens 3, besonders bevorzugt mindestens 10 oder sogar über 100 Attribute für jeden Datenpunkt erfasst. Die Attribute des Originaldatensatzes beinhalten hierbei vorzugsweise sicherheitsrelevante, schützenswerte und/oder personenbezogene Daten, wie beispielsweise personenbezogene Messwerte, Bilddaten, medizinische Daten, medizinische Messwerte usw. Nicht sicherheitsrelevante Attribute sind in den meisten Fällen ebenfalls Teil des Datensatzes

Der Synthesedatensatz im Sinne des erfindungsgemäßen Verfahrens ist ein künstlicher Datensatz, der basierend auf dem Originaldatensatz erzeugt wurde, beispielsweise durch ein Synthesesystem mit dem Ziel, statistische Eigenschaften des Originaldatensatzes widerzuspiegeln.

Sicherheitsrelevante und/oder schützenswerte Daten sind beispielsweise persönliche Daten über Wohnort, Kontaktdaten, Verwandtschaftsverhältnisse, Beruf, Gehalt usw. Auch Bilddaten, Audio- und Videodaten sowie medizinische Daten, wie Blutwerte oder diagnostische Daten, fallen im Allgemeinen unter den Datenschutz und dürfen nicht ohne Schutzmaßnahmen weitergegeben und extern verarbeitet werden.

Häufig ergibt sich die Sensibilität der Daten dabei aus der konkreten Kombination von Attributen, die gemeinsam einer Person oder einem Sachverhalt zugeordnet werden können. Ein einzelnes Attribut, welches beispielsweise einen medizinischen Messwert beinhaltet, wäre unter Datenschutzaspekten weniger kritisch, da über einen einzelnen Messwert üblicherweise keine Zuordnung zu einer Person möglich ist.

Selbst wenn identifizierende Daten, wie Name, Anschrift oder Personalnummer entfernt oder pseudonymisiert werden, so ermöglicht es die Kombination der Attribute teilweise trotzdem, Rückschlüsse auf eine Person zu ziehen. Derartige Datenattribute, aus denen ein Rückschluss auf die Person leicht möglich ist, werden auch als "Quasi-Identifikatoren" bezeichnet.

Bei Meldebehörden, Finanzämtern, Gesundheitseinrichtungen, Krankenkassen werden regelmäßig große Datenmengen erhoben. Diese Datensätze könnten in ihrer Gesamtheit zur Planung von Infrastruktur, medizinischen Kapazitäten usw. verwendet werden. Jedoch sind für statistische Analysen der Daten Rechenkapazitäten und eine entsprechende Ausstattung notwendig, die üblicherweise nicht am selben Ort wie die Originaldaten vorhanden ist. Auch ist es oft von Interesse, Daten mehrerer Anbieter, bzw. Daten, die von unterschiedlichen Stellen erhoben wurden, gemeinsam zu verarbeiten. Aus Gründen der Privatsphäre und des gesetzlichen Datenschutzes, dürfen die Originaldaten aber nicht der Öffentlichkeit oder anderen Anbietern zur Verfügung gestellt werden.

Beispiele für Datensätze, die als Rohdaten nicht unverschlüsselt geteilt werden dürfen, die aber eine hohe Relevanz für die Planung und Optimierung von Infrastruktur und/oder anderen technischen Prozessen haben, sind im Folgenden aufgeführt:

Medizinische Messdaten können beispielsweise Messdaten medizinsicher Geräte, wie Herzschrittmacher, implantierte Defibrillatoren, Langzeit-EKG oder implantierte Blutzuckersensoren umfassen, diese Daten werden in Kombination mit weiteren medizinischen Messdaten, wie Puls, Atmung, Blutdruck etc. erfasst und dürfen aber wegen der nachträglichen Identifizierbarkeit der Patienten nicht als Rohdatensatz geteilt werden. In Form von Synthesedaten, die keine datenschutzverletzenden Daten umfassen, können diese Daten aber von hoher Relevanz für die Planung von medizinischen Kapazitäten und/oder für die Entwicklung verbesserter medizinischer Geräte sein.

Ebenso dürfen Straßenverkehrsdaten und Fahrzeugdaten, die von Fahrzeugen während der Fahrt oder des Parkens gesammelt werden oder die durch Verkehrsüberwachungen erhoben werden, nicht in Rohdatenform geteilt werden. Derartige Daten können Messdaten von Fahrzeugen umfassen, wie beispielsweise GPS-Koordinaten, Akkustand, Verbrauch, Uhrzeit, Temperatur, Geschwindigkeit, Beschleunigung, Beladungsgewicht, Standzeiten etc. Diese Daten können verwendet werden, um Verkehrsflüsse zu simulieren, Unfallschwerpunkte zu identifizieren und Emissionen zu reduzieren. Allerdings sind aus den Rohdaten unter Umständen auch Bewegungen konkreter Fahrzeuge und somit Personen rekonstruierbar, wodurch diese Daten nicht öffentlich geteilt werden dürfen. Ein datenschutzkonformer Synthesedatensatz, der die Eigenschaften der Rohdaten realistisch widerspiegelt, hat somit eine hohe Relevanz für die Infrastrukturplanung und -simulation.

Als weiteres Anwendungsbeispiel kommt die Auswertung von Messdaten von Smart Homes in Betracht. Hierbei werden auch große Mengen technischer Daten gesammelt, beispielsweise Daten zum Energieverbrauch einzelner Komponenten im Smart Home sowie zur Uhrzeit der Nutzung. Diese Daten dürfen nicht öffentlich geteilt werden, sind aber von großer technischer Relevanz für die Planung von Netzkapazitäten etc. Durch die Erstellung von privaten, synthetischen Datensätzen, die die Eigenschaften der gemessenen Smart-Home-Daten realistisch widerspiegeln, ist eine besserer Planung von Netzkapazitäten möglich.

Ebenfalls können technische Protokolldaten (Logs), die Netzwerkdaten von Unternehmen beinhalten zur Planung von Infrastruktur und Netzkapazitäten verwendet werden. Allerdings dürfen auch diese Daten im Allgemeinen aus Sicherheitsgründen nicht öffentlich geteilt werden. Daher besteht auch hier die Möglichkeit, durch einen synthetischen Datensatz die technischen Logdaten von Unternehmen nutzbar für Außenstehende zu machen, wodurch eine verbesserte Planung von Infrastrukturkapazitäten möglich wird. Ein weiterer Anwendungsfall der Weitergabe von Protokolldaten ist die Weiterentwicklung bzw. das Trainieren von Intrusion-Detection-Systemen. Synthetische Datensätze von Protokolldaten unterschiedlicher Unternehmen können konsolidiert werden, um verbesserte Intrusion-Detection-Systeme zu trainieren, wodurch Angriffe auf andere Unternehmen verhindert werden können.

Deshalb besteht ein großer Bedarf an Synthesedaten, die Eigenschaften der Originaldaten möglichst akkurat wiedergeben, aber nicht unter die Datenschutzbestimmungen fallen, so dass sie öffentlich geteilt werden dürfen.

Nach dem Erhalten des Originaldatensatzes und des Synthesedatensatzes umfasst das erfindungsgemäße Verfahren weiterhin das Bestimmen einer Menge kritischer Synthesedatenpunkte durch eine gemeinsame Auswertung einer ersten Evaluierungsfunktion und einer zweiten Evaluierungsfunktion des Originaldatensatzes und Synthesedatensatzes.

Dieser Verfahrensschritt dient der Identifikation von Synthesedatenpunkten, die potenzielle Datenlecks, Sicherheitslücken und/oder Verletzungen der Privatsphäre beinhalten können, sogenannten kritischen Synthesedatenpunkten. Entsprechend der vorliegenden Anmeldung werden kritische Synthesedatenpunkte durch zwei vorzugsweise zunächst getrennt berechnete und anschließend gemeinsam ausgewertete Evaluierungsfunktionen bestimmt. Hierbei werden beide Evaluierungsfunktionen auf dem Originaldatensatz und Synthesedatensatz berechnet, so dass sowohl zumindest eine Teilmenge der Originaldatenpunkte als auch zumindest eine Teilmenge der Synthesedatenpunkte bei der Auswertung berücksichtigt wird.

Nachdem die kritischen Synthesedatenpunkte bestimmt oder identifiziert wurden, beinhaltet das beschrieben Verfahren, als weiteren Schritt das Verändern mindestens eines der kritischen Synthesedatenpunkte.

Durch das Verändern eines oder mehrerer Synthesedatenpunkte können Datenlecks gezielt aus dem Synthesedatensatz entfernt werden. Es wird somit ein modifizierter Synthesedatensatz erstellt, der keine, oder zumindest weniger, privatsphärenverletzende bzw. sicherheitskritische Datenpunkte enthält. Hierdurch wird es ermöglicht, den Synthesedatensatz oder zumindest eine größere Teilmenge des Synthesedatensatzes zur externen Verwendung freizugeben, was wiederum die Nutzung der enthaltenen Daten zu Planungs- und Analysezwecken außerhalb einer sicheren Umgebung ermöglicht. Durch die Möglichkeit zur Nutzung des modifizierten Synthesedatensatzes außerhalb der sicheren Umgebung können die Synthesedaten beispielsweise zur Planung von Infrastruktur, medizinischen Kapazitäten, Produktionskapazitäten usw. verwendet werden.

Die erste Evaluierungsfunktion ist konfiguriert bezüglich einer ersten Abstandsmetrik ein Verhältnis von einem Abstand zwischen jeweils einem Synthesedatenpunkts und mindestens einem benachbarten Originaldatenpunkt zu einer Dichte benachbarter Originaldatenpunkte dieses Synthesedatenpunkts mit einem ersten Schwellwert zu vergleichen

Mit anderen Worten, die erste Evaluierungsfunktion ist implementiert und/oder konfiguriert, bezüglich einer ersten Abstandsmetrik, ein Verhältnis von, einerseits, einem Abstand zwischen jeweils einem Synthesedatenpunkt und mindestens einem benachbarten Originaldatenpunkt und, andererseits, einer Dichte benachbarter Originaldatenpunkte dieses Synthesedatenpunkts zu ermitteln und das ermittelte Verhältnis mit einem ersten Schwellwert, zu vergleichen.

Eine Abstandsmetrik, oder Datenmetrik, ordnet zwei Datensätzen ein numerisches Abstandsmaß bezüglich einer ausgewiesenen statistischen, kombinatorischen oder räumlichen Ausprägung zu. Vorzugsweise befindet sich diese Kennzahl im Einheitsintervall [0, 1], wobei die Randwerte Null und Eins eine sehr hohe bzw. geringe Ähnlichkeit der evaluierten Datensätze ausdrücken. Ein Anwendungsfall besteht darin, einen vorgegebenen Originaldatensatz mit einem Synthesedatensatz vergleichend zu untersuchen.

Grundsätzlich unterscheidet man bei Datenmetriken zwischen numerischen und kategoriellen Einträgen in Datensätzen. Eine Metrik zur Bestimmung von Ähnlichkeiten und Abständen bei Datenpunkten, die sowohl numerische als auch kategorielle Attribute enthalten, ist die sogenannte Gower-Distanz.

Für numerische Attributwerte $x_i$ und $x_i'$ kann eine Gower-Ähnlichkeit wie folgt definiert werden:

$$s(x_i, x_i') := 1 - \frac{|x_i - x_i'|}{N}$$

mit Normalisierungskoeffizient N als größte auftretende Distanz. Sind $x_i$ und $x_i'$ kategorielle Attributwerte, so wird die Gower-Ähnlichkeit wie folgt definiert:

$$s(x_i, x_i') := \begin{cases} 1, & \text{falls } x_i = x_i', \\ 0, & \text{sonst.} \end{cases}$$

Zwei Datenpunkte $x \in D$ und $x' \in D'$ derselben Länge p können dann mittels der Gower-Ähnlichkeit verglichen werden:

$$S_{Gower}(x, x') := \frac{\sum_{i=1}^{p} s(x_i, x_i')}{p}$$

Die Gower-Distanz zwischen x und x' ist dann gegeben durch:

$$d_{Gower}(x, x') := \sqrt{1 - S_{Gower}(x, x')}$$

Auf Basis der geringsten Distanz der obigen Distanzmatrix lässt sich erkennen, welche Punkte des synthetischen Datensatz eine geringe Distanz zum Originaldatensatz aufweisen. Gegebenenfalls können dadurch auch Duplikate (Abstand Null) erkannt werden.

Alternative Metriken zur Berechnung von Abständen bei numerischen Datenpunkten sind ein euklidischer Abstand oder ein Manhattan-Abstand. Im Allgemeinen kann auch für jeden Attributtyp ein Abstandsmaß definiert werden, welches dann über die Menge der Attribute gemittelt eine Abstandsberechnung für zwei Datenpunkte ermöglicht. Hierbei können Abstände beispielsweise zusätzlich normiert und/oder gewichtet werden.

Basierend auf einem Abstandsmaß kann beispielsweise eine Abstandsmatrix zwischen zwei Datensätzen bestimmt werden. Ein Abstandsmaß und/oder eine Abstandsmatrix kann zur Grundlage für eine Bestimmung einer Datenpunktdichte in einem Datensatz genommen werden.

Für die Bewertung der Privatsphäre bzw. der Sicherheit des Synthesedatensatzes kann beispielsweise eine lokale Originalpunktdichte relevant sein, d.h. die Dichte der Originaldatenpunkte, die dem jeweiligen Synthesedatenpunkt bezüglich des Abstandsmaßes am nächsten liegen. Als ein Maß für die Dichte kann hierbei ein Abstandsverhältnis der nächsten Nachbarn (Nearest Neighbor Distance Ratio, NNDR) betrachtet werden.

Für einen Datenpunkt Y aus dem Synthesedatensatz ist die Nearest Neighbor Distance Ratio gegeben durch den Quotienten:

$$\frac{\textit{Abstand zum nächsten Nachbarn X1 des Originaldatensatzes}}{\textit{Abstand zum zweitnächsten Nachbarn X2 des Originaldatensatzes}}$$

Im obigen Bruch kann das zugrundeliegende Abstandsmaß frei gewählt werden. Beispielsweise kann auch die Gower-Distanz verwendet werden. Außerdem kann der zweitnächste Nachbar durch den n-nächsten Nachbarn oder durch einen Mittelwert der Abstände der m nächsten Nachbarn ersetzt werden.

Die erste Evaluierungsfunktion analysiert somit vorzugsweise das Verhältnis von Distanz und Dichte, im Folgenden auch als Distanz-Dichte-Verhältnis bezeichnet, zwischen Datenpunkten des Synthesedatensatzes und Datenpunkten des Originaldatensatzes, um auf diese Art und Weise potenzielle Datenlecks oder einen potenziellen Mangel an Privatheit der Synthesedaten zu identifizieren. Beispielsweise können somit Synthesedatenpunkte mit geringem Abstand zu einzelnen Originaldatenpunkten in Regionen geringer Originaldatenpunktdichte als potenzielles Datenleck identifiziert werden, da diese Eigenschaften auf einen zumindest teilweise reproduzierten Datenpunkt hinweisen können. Mit einem reproduzierten Datenpunkt ist hierbei ein Originaldatenpunkt gemeint, der identisch, fast identisch oder bezüglich einer Kombination von Attributwerten identisch zu einem Synthesedatenpunkt ist. Dies ist ein Hinweis auf ein "Merken" oder "Erlernen" einer konkreten Merkmalskombination durch das verwendete Synthesesystem.

Wenn beispielsweise ein Synthesedatenpunkt nur eine geringe Distanz zu einem Originaldatenpunkt aufweist, jedoch einen hohen NNDR, so impliziert dies, dass es sich um ein Cluster im Originaldatensatz handelt und daher nur ein geringer Privatsphäreverlust erwartbar ist.

Sind jedoch sowohl die Distanz als auch die Dichte zum nächsten Originaldatenpunkt klein, so handelt es sich um die Synthese eines Ausreißers und die Zeile sollte ggf. aus dem synthetisch erzeugten Datensatz entfernt werden.

Die zweite Evaluierungsfunktion ist hierbei implementiert und/oder konfiguriert für mindestens eine seltene Attributkombination des Originaldatensatzes eine Randverteilung dieser seltenen Attributkombination des Synthesedatensatzes zu bestimmen und mit mindestens einem zweiten Schwellwert zu vergleichen.

Die zweite Evaluierungsfunktion bestimmt weitere potenzielle Datenlecks anhand von statistischen Tests in Form von der Auswertung der Randverteilungen der Wahrscheinlichkeitsverteilung einer Vielzahl an verschiedenen Attributkombinationen von Synthesedatensatz und/oder Originaldatensatz. Eine Attributkombination im Sinne dieser Anmeldung kann ein oder mehrere Attribute umfassen. Es können vorzugsweise einzigartige oder statistisch seltene Attributkombinationen im Originaldatensatz identifiziert werden. Für diese seltenen Attributkombinationen können dann die Randverteilungen, also die Verteilungen, bei denen diese Attributwerte fixiert werden, analysiert werden. Hierbei kann vorzugsweise in den Randverteilungen nach einer Duplizierung der seltenen Attributkombination, beziehungsweise nach einer statistisch unwahrscheinlichen Häufung seltener Attributkombinationen, gesucht werden, um somit potenzielle Datenlecks zu identifizieren. Die zweite Evaluierungsfunktion hat den zusätzlichen Effekt, dass mit ihr beispielsweise reproduzierte prägnante Attributkombinationen in Regionen mit durchschnittlicher oder hoher Datendichte identifiziert werden können, die durch die erste Evaluierungsfunktion evtl. nicht als potenzielle Datenlecks identifiziert werden würden. Auch können mit der zweiten Evaluierungsfunktion möglicherweise vor allem Datenlecks erkannt werden, bei denen ein Datenpunkt des Originaldatensatzes nur teilweise reproduziert wurde.

Um eine Randverteilung mit einem Schwellwert zu vergleichen kann beispielsweise ein charakteristischer Wert der Randverteilung, wie Mittelwert, Median, Varianz, Standardabweichung, Maximum etc. verwendet werden oder der Schwellwert kann in Form einer Schwellwertverteilung vorliegen, so dass ein Vergleich zwischen einer Randverteilung und der Schwellwertverteilung beispielsweise in Form eines Integrals über die Abstandsfläche zwischen den beiden Verteilungen ermöglicht wird. Beispielsweise kann die Randverteilung mit der Wahrscheinlichkeit für das zufällige Auftreten der jeweiligen Attributkombination verglichen werden, so dass diese Wahrscheinlichkeit dann als Schwellwert dient.

Eine kombinierte Auswertung der ersten und zweiten Evaluierungsfunktion kann dann für zumindest eine Teilmenge der Synthesedatenpunkte, vorzugsweise für alle Synthesedatenpunkte, bestimmen, ob einzelne Synthesedatenpunkte ein Sicherheitsrisiko oder potenzielles Datenleck darstellen. Diese Synthesedatenpunkte können dann als sogenannte kritische Synthesedatenpunkte identifiziert werden.

Eine gemeinsame Auswertung der ersten und zweiten Evaluierungsfunktion kann beispielsweise erfolgen, indem Synthesedatenpunkte dann als kritische Synthesedatenpunkte bestimmt werden, wenn sie anhand der ersten Evaluierungsfunktion oder anhand der zweiten Evaluierungsfunktion als kritische Synthesedatenpunkte bestimmt werden. Alternativ oder zusätzlich, können Synthesedatenpunkte dann als kritische Synthesedatenpunkte bestimmt werden, wenn sie zunächst durch eine der Evaluierungsfunktionen als potenziell kritische Synthesedatenpunkte bestimmt werden und dann die potenziell kritischen Synthesedatenpunkte anschließend durch die jeweils andere Evaluierungsfunktion als kritische Synthesedatenpunkte verifiziert werden. Bei einer derartigen mehrschrittigen Evaluierung können beispielsweise die zu Grunde liegenden Datensätze für die als zweites angewandte Evaluierungsfunktion verändert, beispielsweise reduziert, werden oder die Parameter der als zweites angewandten Evaluierungsfunktion können basierend auf dem Ergebnis der zuerst angewandten Evaluierungsfunktion angepasst werden.

Das beschriebene Verfahren hat den technischen Effekt, dass Datenlecks im Synthesedatensatz anhand der kombinierten Auswertung der beiden Evaluierungsfunktionen identifiziert werden können und dass durch die beiden Evaluierungsfunktionen verschiedene Arten von Datenlecks, die jeweils durch eine einzelne Evaluierungsfunktion nicht identifiziert werden würden, identifiziert werden können.

Weiterhin hat das Verfahren den Effekt, dass Datenpunkte des Synthesedatensatzes selektiv modifiziert werden können, um Datenlecks zu entfernen und eine Sicherheit und Anonymität des Datensatzes sicher zu stellen. Die Anonymisierung eines Datensatzes kann hierbei als eine Art Verschleierung der Originaldaten verstanden werden, die es Dritten ermöglicht, den resultierenden modifizierten Datensatz zu verwenden, allerdings ohne, dass besagte Dritte Zugriff auf sensible Daten bzw. seltene oder einzigartige Attributwertskombinationen des Originaldatensatzes erlangen.

Nach dem Verändern mindestens eines der kritischen Synthesedatenpunkte durch das oben beschriebene Verfahren ist es optional möglich, das Verfahren basierend auf dem Originaldatensatz und dem veränderten Synthesedatensatz, welcher die veränderten Synthesedatenpunkte umfasst, zu wiederholen. Hierbei wird optional der oben beschriebene Schritt des Bestimmens einer Menge von kritischen Synthesedatenpunkten aus der Vielzahl der Synthesedatenpunkte, basierend auf einer erneuten gemeinsamen Auswertung der ersten Evaluierungsfunktion und der zweiten Evaluierungsfunktion, wiederholt.

Wenn bei der wiederholten bzw. erneuten Bestimmung kritischer Synthesedatenpunkte weiterhin Synthesedatenpunkte als kritisch bestimmt werden, d.h. wenn die Menge der kritischen Synthesedatenpunkte bei der wiederholten Ausführung der Bestimmung kritischer Synthesedatenpunkte nicht leer ist, so kann der oben beschriebene Schritt des Veränderns von mindestens einem der kritischen Synthesedatenpunkte optional ebenfalls wiederholt werden. Anschließend kann die beschriebene Schleife optional erneut ausgeführt werden, bis die Menge der bestimmten kritischen Synthesedatenpunkte entweder leer ist oder eine andere Abbruchbedingung erreicht wurde. Als weitere Abbruchbedingung für die Ausführung der Schleife kann beispielsweise eine maximale Anzahl an Schleifendurchläufen oder ein zeitliches Limit verwendet werden.

Falls nach einer maximalen Anzahl an Schleifendurchläufen weiterhin kritische Synthesedatenpunkte im Synthesedatensatz vorhanden sind, so kann optional ein neuer Synthesedatensatz basierend auf dem Originaldatensatz generiert oder anderweitig erhalten werden. Mögliche Ausführungsformen für die Generierung eines Synthesedatensatzes sind weiter unten beschrieben.

Beispielsweise kann das Verändern eines kritischen Synthesedatenpunkts verschiedene Optionen umfassen. Der kritische Synthesedatenpunkt kann beispielsweise aus dem Synthesedatensatz gelöscht bzw. entfernt werden. Dies hat den Vorteil, dass durch diesen Datenpunkt dann keinerlei privatsphäreverletzende Attributwerte mehr preisgegeben werden können.

Alternativ kann der kritische Synthesedatenpunkt verändert werden, indem mindestens ein Synthesewert eines Attributs des kritischen Synthesedatenpunkts verändert wird. Dies hat den Vorteil, dass der kritische Synthesedatenpunkt an sich im Synthesedatensatz verbleiben kann, jedoch die privatsphäreverletzenden Synthesewerte der Attribute verändert werden. Alternativ ist es auch möglich, die Synthesewerte aller Attribute des Synthesedatenpunkts zu verändern. Bei der Veränderung der Synthesewerte von einzelnen oder allen Attributen des Synthesedatenpunkts, können die einzelnen Synthesewerte beispielsweise verrauscht werden, beispielsweise indem die numerischen Werte um einen zufällig bestimmten Rauschwert verändert werden.

Es ist weiterhin oder alternativ auch möglich, den Synthesedatensatz durch eine erneute Synthese vollständig zu ersetzen. Hierbei könnten beispielsweise Parameter des Synthesesystems verändert werden, mit dem Ziel, dass der neue Synthesedatensatz weniger kritische Synthesedatenpunkte enthalten soll. Da dies jedoch bei der Datensynthese üblicherweise nicht direkt gesteuert werden kann, würden vorzugsweise nach der erneuten Synthese des neuen Synthesedatensatzes, wiederum durch das beschriebene Verfahren kritische neue Synthesedatenpunkte identifiziert und selektiv modifiziert werden.

Nachdem ein oder mehrere der kritischen Synthesedatenpunkte verändert wurden, kann vorzugsweise das beschriebene Verfahren erneut ausgeführt werden, um zu überprüfen ob weiterhin kritische Synthesedatenpunkte vorhanden sind.

Vorzugsweise umfassen die Attribute der Originaldatenpunkte zumindest eine Teilmenge der folgenden Datenarten: persönliche Daten wie Größe, Gewicht, ethnische Zugehörigkeit, Adressdaten, Bilddaten, GPS-Daten, Bewegungsprofile, medizinische Daten und/oder Blutwerte. Somit enthalten die Originaldaten vorzugsweise Messdaten, die jeweils einen engen Bezug zu der Privatsphäre einer Person aufweisen. Die Attribute der Synthesedatenpunkte umfassen vorzugsweise zumindest eine Teilmenge der Attribute der Originaldatenpunkte. Beispielsweise können die Synthesedatenpunkte alle Attribute der Originaldatenpunkte enthalten oder alle Attribute der Originaldatenpunkte außer dem Namen (oder anderem eindeutigen Identifikator) der durch den jeweiligen Originaldatenpunkt repräsentierten Person.

Vorzugsweise ist es möglich, dass die erste Evaluierungsfunktion bei Eingabe eines Synthesedatenpunkts einen ersten Evaluierungswert bestimmt, wobei der erste Evaluierungswert beispielsweise ein numerischer Wert ist, der reellwertig sein kann oder alternativ ein zwischen 0 und 1 normierter Wert sein kann, wobei die Normierung beispielsweise durch eine Normalverteilung oder anhand des größten Werts der aktuellen Auswertung erfolgen kann.

Weiterhin ist es möglich, dass die zweite Evaluierungsfunktion bei Eingabe eines Synthesedatenpunkts einen zweiten Evaluierungswert bestimmt, wobei der zweite Evaluierungswert beispielsweise ein numerischer Wert ist, der reellwertig sein kann oder alternativ ein zwischen 0 und 1 normierter Wert sein kann, wobei die Normierung beispielsweise durch eine Normalverteilung oder anhand des größten Werts der aktuellen Auswertung erfolgen kann.

Beispielsweise kann so ein Synthesedatenpunkt als kritischer Synthesedatenpunkt bestimmt werden, wenn die erste Evaluierungsfunktion bezüglich dieses Synthesedatenpunkts, beispielsweise in Form des ersten Evaluierungswerts des Synthesedatenpunkts, oberhalb des ersten Schwellwert und/oder die zweite Evaluierungsfunktion bezüglich dieses Synthesedatenpunkts, beispielsweise in Form des zweiten Evaluierungswerts des Synthesedatenpunkts, oberhalb des zweiten Schwellwerts liegt.

Die Verwendung von Evaluierungsfunktionen die als Ausgaben numerische Werte liefern, hat den Vorteil, dass damit ein Maß für die Privatheit des Synthesedatensatzes bestimmbar ist und ein einfacher Mechanismus zur objektiven Bestimmung von kritischen Synthesedatenpunkten möglich ist.

Ein Synthesedatenpunkt kann somit basierend auf der ersten und/oder zweiten Evaluierungsfunktion als kritischer Synthesedatenpunkt identifiziert werden. Alternativ kann ein Synthesedatenpunkt als kritischer Synthesedatenpunkt identifiziert werden, wenn sowohl die erste als auch die zweite Evaluierungsfunktion oberhalb der jeweiligen Schwellwerte liegen.

Die Schwellwerte können hierbei beispielsweise vorab festgelegt werden, beispielsweise basierend auf Erfahrungswerten oder basierend auf einer Vorhersage guter Schwellwerte basierend auf künstlicher Intelligenz, Datenanalyse oder ähnlichen Verfahren.

Alternativ ist es auch möglich, dass der Schwellwert basierend auf einer dynamischen Analyse der vorliegenden Original- und/oder Synthesedatensätze festgelegt wird. Beispielsweise ist es möglich, den ersten und zweiten Evaluierungswert jeweils für zumindest eine Teilmenge der Synthesedatenpunkte zu bestimmen und die bestimmten Evaluierungswerte dann zu clustern und einen jeweiligen Schwellwert dynamisch und automatisch so zu bestimmen, dass der jeweilige Evaluierungswert bei statistischen Ausreißern oberhalb des jeweiligen Schwellwerts liegt.

Alternativ oder zusätzlich ist es auch möglich, das Bestimmen, dass ein Synthesedatenpunkt ein kritischer Synthesedatenpunkt ist, durch ein mehrschrittiges Bestimmen der beiden Evaluierungsfunktionen durchzuführen.

Beispielsweise kann zunächst bestimmt werden, dass die erste Evaluierungsfunktion bezüglich dieses Synthesedatenpunkts oberhalb des ersten Schwellwerts liegt und danach können beispielsweise nachfolgend Randverteilungen von Attributkombinationen dieses Synthesedatenpunkts bestimmt werden. Diese Randverteilungen bzw. aus den jeweiligen Randverteilungen bestimmte charakteristische Werte, können dann mit entsprechenden Schwellwerten verglichen werden.

Alternativ oder zusätzlich ist es auch möglich, einen Synthesedatenpunkt als kritischen Synthesedatenpunkt zu bestimmen, indem zunächst bestimmt wird, dass die zweite Evaluierungsfunktion bezüglich einer Attributkombination der Synthesedatenpunkte oberhalb des zweiten Schwellwerts liegt. In diesem Fall kann dann beispielsweise nachfolgend die erste Evaluierungsfunktion für diesen Synthesedatenpunkt nochmal basierend auf den auf die auffällige Attributkombination eingeschränkten Original- und Synthesedatendatensätzen bestimmt werden.

Somit ist eine kombinierte Auswertung oder Evaluierung anhand der beiden verwendeten Evaluierungsfunktionen möglich.

Es ist optional möglich, als weiteren Verfahrensschritt den ersten Schwellwert basierend auf dem Originaldatensatz und/oder Synthesedatensatz zu bestimmen. Weiterhin ist es ebenfalls optional möglich, in einem weiteren Verfahrensschritt den zweiten Schwellwert basierend auf dem Originaldatensatz und/oder Synthesedatensatz zu bestimmen.

Eine Bestimmung des ersten und/oder zweiten Schwellwerts basierend auf dem Originalund/oder Synthesedatensatz ermöglicht eine passgenaue Analyse und Modifikation des Synthesedatensatzes.

Es ist optional weiterhin möglich, bei einer wiederholten Ausführung des Verfahrens einen oder beide Schwellwerte jeweils neu zu berechnen. Dadurch ist es beispielsweise möglich, zunächst die "kritischsten" Synthesedatenpunkte zu identifizieren und zu verändern und erst anschließend, basierend auf anderen Schwellwerten, die weiteren kritischen Synthesedatenpunkte zu identifizieren und zu verändern. Dadurch kann beispielsweise verhindert werden, dass weniger kritische (basierend auf den jeweiligen Evaluierungsfunktionen) Synthesedatenpunkte durch kritischere Synthesedatenpunkte überlagert und somit fälschlicherweise nicht erkannt werden.

Das Verfahren kann weiterhin optional, vor dem Erhalten des Synthesedatensatzes, einen weiteren Schritt umfassen, in dem der Synthesedatensatz basierend auf dem Originaldatensatz generiert wird.

Ein Synthesedatensatz kann beispielsweise unter Verwendung von maschinellem Lernen generiert werden, vorzugsweise ist es möglich hierzu ein generatives ML-Model zu verwenden. Hierzu wird zunächst ein Modell des maschinellen Lernens basierend auf dem Originaldatensatz trainiert. Dazu können insbesondere ein oder mehrere Modelle des maschinellen Lernens (auch als ML-Modelle bezeichnet) mittels eines unbeaufsichtigten (engl. unsupervised) Lernverfahrens trainiert werden, um Daten entsprechend den Eigenschaften des Originaldatensatzes zu generieren. Hierzu können beispielsweise ML-Modelle der folgenden Arten zum Einsatz kommen: Variational Autoencoder (VAE), Generative Adversarial Network (GAN), Private Data Release mittels Bayes'schen Netzen (PrivBayes). Auch andere ML-Modelle, die zur Datengenerierung geeignet sind, können verwendet werden. Optional kann vor dem Trainingsschritt eine Vorverarbeitung der Originaldaten stattfinden, bei der die Originaldaten beispielsweise normalisiert oder gefiltert werden.

Durch unterschiedliche Parameter und Randomisierung während der Synthese, werden üblicherweise bei zwei Durchläufen einer Datensynthese basierend auf demselben Originaldatensatz zwei (leicht) unterschiedliche Synthesedatensätze erzeugt.

Alternativ kann ein Synthesedatensatz auch durch Samplen der Verteilungen der unterschiedlichen Attribute des Originaldatensatzes erfolgen.

Weiterhin kann das beschriebene Verfahren als weiteren optionalen Schritt umfassen: Wenn keine kritischen Synthesedatenpunkte bestimmt werden, erfolgt die Übermittlung der Synthesedaten an einen externen Benutzer.

Der externe Benutzer kann hierbei vorzugsweise ein externer Computer, Server, Rechenzentrum oder ähnliches sein. Die Übermittlung an externe Benutzer ermöglicht eine weitergehende externe Bearbeitung und Verarbeitung der Synthesedaten. Beispielsweise können die Synthesedaten zur statistischen Analyse der Originaldaten verwendet werden, da die Synthesedaten, trotz anonymisierender Abweichungen der einzelnen Attributwerte der einzelnen Synthesedatenpunkte, weiterhin statistische Eigenschaften des Originaldatensatzes aufweisen. Somit können Synthesedaten die basierend auf medizinischen Originaldaten erstellt wurden, durch den externen Benutzer beispielsweise zur Bestimmung von medizinischen Kapazitäten, zur Vorhersage und nachfolgender Produktion von benötigten medizinischen Verbrauchsartikeln usw. verwendet werden.

Auch in anderen Branchen können basierend auf Synthesedaten beispielsweise Infrastrukturprojekte oder Produktionskapazitäten von Verbrauchsgütern bestimmt werden.

Ein weiterer Aspekt der Erfindung betrifft eine Datenverarbeitungsvorrichtung zur selektiven Veränderung eines Synthesedatensatzes. Die Datenverarbeitungsvorrichtung ist beispielsweise ein Computer, ein Server oder ein Computersystem und umfasst zumindest einen Prozessor, einen Datenspeicher und einen Instruktionsspeicher.

Der Datenspeicher ist dabei konfiguriert zum Speichern eines Originaldatensatzes und eines basierend auf dem Originaldatensatzes generierten Synthesedatensatzes. Der Originaldatensatz umfassend eine Vielzahl von Originaldatenpunkten, wobei jeder der Originaldatenpunkte Originalwerte für eine Vielzahl von Attributen umfasst. Der Synthesedatensatz umfassend eine Vielzahl von Synthesedatenpunkten, wobei jeder der Synthesedatenpunkte Synthesewerte für eine Vielzahl der Attribute umfasst.

Der Instruktionsspeicher ist hierbei konfiguriert zum Speichern von Programminstruktionen, die wenn sie auf der Datenverarbeitungsvorrichtung ausgeführt werden, die Datenverarbeitungsvorrichtung veranlassen das folgende Verfahren auszuführen.

- Bestimmen einer Menge kritischer Synthesedatenpunkte durch eine gemeinsame Auswertung einer ersten Evaluierungsfunktion und einer zweiten Evaluierungsfunktion und

- Verändern mindestens eines der kritischen Synthesedatenpunkte.

Die erste und zweite Evaluierungsfunktionen sind hierbei, wie in Bezug auf das Verfahren bereits diskutiert, definiert.

Somit vergleicht die erste Evaluierungsfunktion bezüglich einer ersten Abstandsmetrik ein Verhältnis von einem Abstand zwischen jeweils einem Synthesedatenpunkt und mindestens einem benachbarten Originaldatenpunkt zu einer Dichte benachbarter Originaldatenpunkte dieses Synthesedatenpunkts mit einem ersten Schwellwert.

Die zweite Evaluierungsfunktion vergleicht für mindestens eine seltene Attributkombination des Originaldatensatzes eine Randverteilung dieser seltenen Attributkombination des Synthesedatensatzes mit mindestens einem zweiten Schwellwert.

Der Prozessor kann weiterhin konfiguriert sein, die oben im Zusammenhang mit dem computerimplementierten Verfahren beschriebenen optionalen Schritte auszuführen.

Ein weiterer Aspekt der Erfindung betrifft ein Computersystem zur Distribution eines Synthesedatensatzes. Das Computersystem umfasst hierbei eine erste Datenverarbeitungsvorrichtung zur selektiven Veränderung eines Synthesedatensatzes, wie oben beschrieben, wobei die erste Datenverarbeitungsvorrichtung innerhalb einer sicheren Umgebung angeordnet ist. Weiterhin umfasst das Computersystem eine zweite Datenverarbeitungsvorrichtung, die außerhalb der sicheren Umgebung angeordnet ist. Die erste Datenverarbeitungsvorrichtung ist konfiguriert, den veränderten Synthesedatensatz an die zweite Datenverarbeitungsvorrichtung zu übertragen.

Vorzugsweise ist die erste Datenverarbeitungsvorrichtung weiterhin konfiguriert, vor der Übertragung des veränderten Synthesedatensatzes an die zweite Datenverarbeitungsvorrichtung erneut eine Menge kritischer Synthesedatenpunkte mittels einer gemeinsamen Auswertung der ersten und zweiten Evaluierungsfunktion zu bestimmen und den veränderten Synthesedatensatz nur dann an die zweite Datenverarbeitung zu übertragen, falls bei der erneuten Bestimmung kritischer Synthesedatenpunkte eine leere Menge kritischer Synthesedatenpunkte bestimmt wurde. In anderen Worten, der veränderte Synthesedatensatz darf optional nur dann oder erst dann an die zweite Datenverarbeitungsvorrichtung außerhalb der sicheren Umgebung übertragen werden, wenn keiner kritischen Synthesedatenpunkte mehr im Synthesedatensatz vorhanden ist.

Dadurch wird die externe Übertragung und Nutzung der Synthesedaten ermöglicht und es wird gleichzeitig sichergestellt, dass keine sicherheitskritischen und/oder privatsphärenverletzenden Daten außerhalb der sicheren Umgebung übertragen werden können.

In einem weiteren Aspekt umfasst die vorliegende Erfindung ein Computerprogrammprodukt, welches Befehle umfasst, die bei der Ausführung des Programms durch einen Computer, diesen veranlassen die Schritte des oben beschriebenen Verfahrens auszuführen.

Die Erfindung betrifft somit ein computerimplementiertes Verfahren und eine Datenverarbeitungsvorrichtung zur selektiven Veränderung eines Synthesedatensatzes. Das Verfahren umfasst das Erhalten eines Originaldatensatzes, umfassend eine Vielzahl von Originaldatenpunkten, und eines basierend auf dem Originaldatensatz synthetisierten Synthesedatensatzes, umfassend eine Vielzahl von Synthesedatenpunkten. Das Verfahren umfasst weiterhin ein Bestimmen einer Menge kritischer Synthesedatenpunkte aus der Vielzahl der Synthesedatenpunkte durch eine gemeinsame Auswertung einer ersten Evaluierungsfunktion und einer zweiten Evaluierungsfunktion des Originaldatensatzes und Synthesedatensatzes, und ein Verändern mindestens eines der kritischen Synthesedatenpunkte.

Grundsätzlich wird angemerkt, dass alle Merkmale, die in Bezug auf bestimmte Aspekte oder Ausführungsformen der Erfindung offenbart werden, auch mit anderen Aspekten oder Ausführungsformen der Erfindung technisch sinnvoll kombinierbar sind. Dies gilt auch über unterschiedliche technische Gegenstände und Gegenstandskategorien hinweg. Insbesondere gilt dies auch auszugsweise für einzelne Merkmale, solange hierin nicht explizit darauf hingewiesen wird oder es durch einen technischen Widerspruch offensichtlich ist, dass zwischen bestimmten Merkmalen ein untrennbarer funktional-technischer Zusammenhang besteht, der zur Ausführung der Erfindung beibehalten werden muss.

Im Folgenden wird die Erfindung anhand von Ausführungsbeispielen und deren skizzenhafter Darstellung erläutert. Hierbei zeigen:

Figur 1     ein Blockschema der beschrieben Datenverarbeitungsvorrichtung und des beschriebenen Computersystems;

Figur 2     ein Ablaufdiagramm einer Ausführungsform des beschriebenen Verfahrens;

Figur 3     ein Ablaufdiagramm einer weiteren Ausführungsform des beschriebenen Verfahrens;

Figur 4A     eine grafische Darstellung von exemplarischen Originaldaten und Synthesedaten;

Figur 4B     eine grafische Darstellung des Distanz-Dichte-Verhältnisses der Daten aus Figur 4A;

Figur 4C     eine grafische Darstellung der kritischen Synthesedatenpunkte aus Figur 4A.

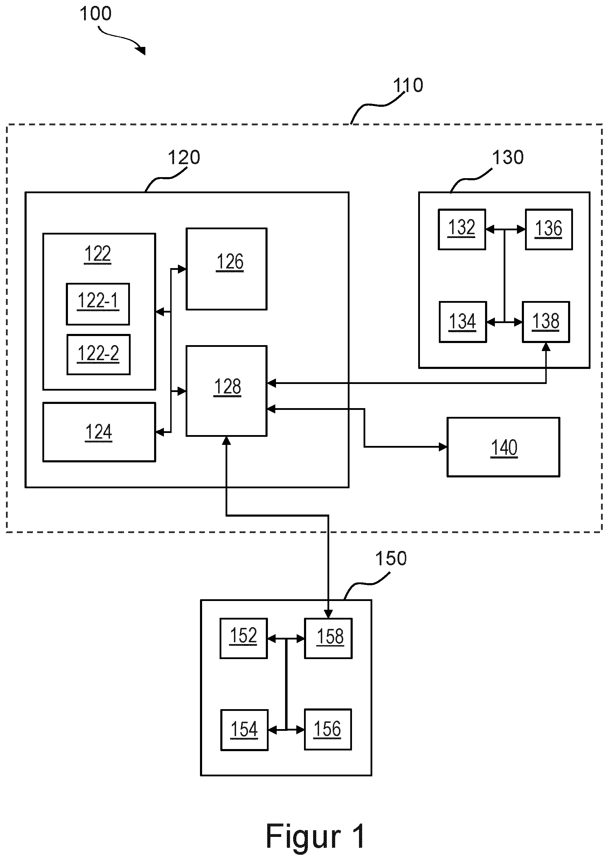

Figur 1 zeigt eine schematische Darstellung der erfindungsgemäßen Datenverarbeitungsvorrichtung 120 im Kontext eines Computersystems 100.

Die Bestandteile der Datenverarbeitungsvorrichtung 120 beinhalten beispielsweise zumindest einen oder mehrere Prozessoren 126 oder Berechnungsvorrichtungen, eine Speichervorrichtung, wobei die Speichervorrichtung einen Datenspeicher 122 und einen Instruktionsspeicher 124 umfasst, sowie ein Bussystem, das die verschiedenen Bestandteile mit dem Prozessor 126 verbindet.

Der Datenspeicher 122 speichert einen Originaldatensatz 122-1 und einen Synthesedatensatz 122-2, der basierend auf dem Originaldatensatz 122-1 generiert wurde.

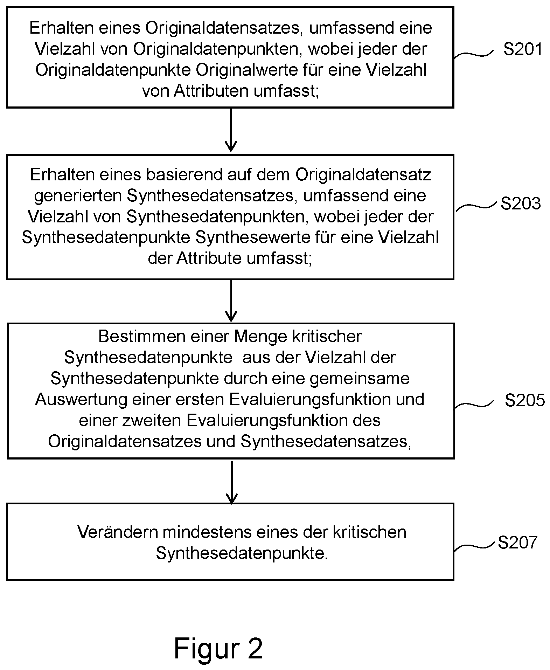

Der Instruktionsspeicher 124 umfasst den Programmcode, der bei Ausführung auf dem Prozessor 126 der Datenverarbeitungsvorrichtung 120 die Datenverarbeitungsvorrichtung 120 veranlasst das Verfahren aus Figur 2 auszuführen.

Die Datenverarbeitungsvorrichtung 120 kann außerdem mit externen Geräten, auch als Peripheriegeräte 140 bezeichnet, kommunizieren, beispielsweise mit Ein- und/oder Ausgabegeräten, wie zum Beispiel einer Tastatur, Maus, Touch-Screen, Monitor, Display etc. Zur Kommunikation mit externen Geräten beinhaltet die Datenverarbeitungsvorrichtung 120 weiterhin eine Kommunikationsschnittstelle 128.

Die Datenverarbeitungsvorrichtung 120 befindet sich in einer sicheren Umgebung 110, die z.B. durch eine Firewall vor externen Zugriffen geschützt wird. Innerhalb dieser sicheren Umgebung 110 ist die Speicherung und Verarbeitung von sensiblen Daten aus dem Originaldatensatz 122-1 erlaubt.

Die Datenverarbeitungsvorrichtung 120 kann aber, mittels der Kommunikationsschnittstelle 128, mit Peripheriegeräten 140 und mit externen Benutzern oder externe Datenverarbeitungsvorrichtungen 130, 150 kommunizieren.

Die in Figur 1 gezeigten externen Datenverarbeitungsvorrichtungen 130, 150 beinhalten vorzugsweise ebenfalls jeweils einen Prozessor 136, 156, einen Datenspeicher 132, 152, einen Instruktionsspeicher 134, 154 sowie jeweilige Kommunikationsschnittstellen 138, 158, über die die jeweilige externe Datenverarbeitungsvorrichtung 130, 150 mit der Datenverarbeitungsvorrichtung 120 kommunizieren und Daten austauschen kann.

Bei externen Datenverarbeitungsvorrichtungen 130, 150 und ebenfalls bei externen Benutzern ist zwischen sicheren externen Datenverarbeitungsvorrichtungen 130, die sich innerhalb der sicheren Umgebung 110 befinden, und unsicheren externen Datenverarbeitungsvorrichtungen 150, die sich außerhalb der sicheren Umgebung 110 befinden, zu unterscheiden.

Ob eine externe Datenverarbeitungsvorrichtung sich innerhalb oder außerhalb der sicheren Umgebung befindet, macht einen Unterschied, insbesondere bei der Art der Daten, die über die Schnittstelle 128 an die jeweiligen externen Datenverarbeitungsvorrichtung 130, 150 übertragen werden dürfen. Während eine Übertragung von Daten von der Datenverarbeitungsvorrichtung 120 an sichere externe Datenverarbeitungsvorrichtungen oft unbeschränkt möglich ist, sind nur bestimmte Daten zur externen Übertragung an externe Benutzer oder Datenverarbeitungsvorrichtungen 150, die sich außerhalb der sicheren Umgebung 110 befinden, freigegeben.

Synthesedaten 122-2 dürfen, im Gegensatz zu Originaldaten 122-1, prinzipiell extern übertragen werden, aber für eine externe Übertragung muss sichergestellt sein, dass die Synthesedaten 122-2 keine Datenlecks oder Sicherheitsrisiken enthalten. Ein Datenleck besteht immer dann, wenn aus den Synthesedaten 122-2 Rückschlüsse auf schützenswerte Zusammenhänge der Originaldaten gezogen werden können. Beispielsweise wenn bei personenbezogenen Daten, wie medizinischen Patientendaten, die Attributkombinationen eine Identifikation dieser Person möglich oder wahrscheinlich machen.

Alternativ kann sich die Datensynthese, wie oben bereits beschrieben, auf medizinische Messdaten, Straßenverkehrsdaten, Fahrzeugdaten, Messdaten von Smart Homes oder technische Protokolldaten beziehen.

Somit ist es erforderlich, Synthesedaten 122-2 vor ihrer Übermittlung an externe Benutzer auf ihre tatsächliche Sicherheit oder Privatheit, bzw. auf einen Mangel an Privatheit in Form von Datenlecks, zu überprüfen. Gegebenenfalls müssen die Synthesedaten 122-2 dann noch stärker anonymisiert werden, bevor sie an externen Benutzer übertragen werden können.

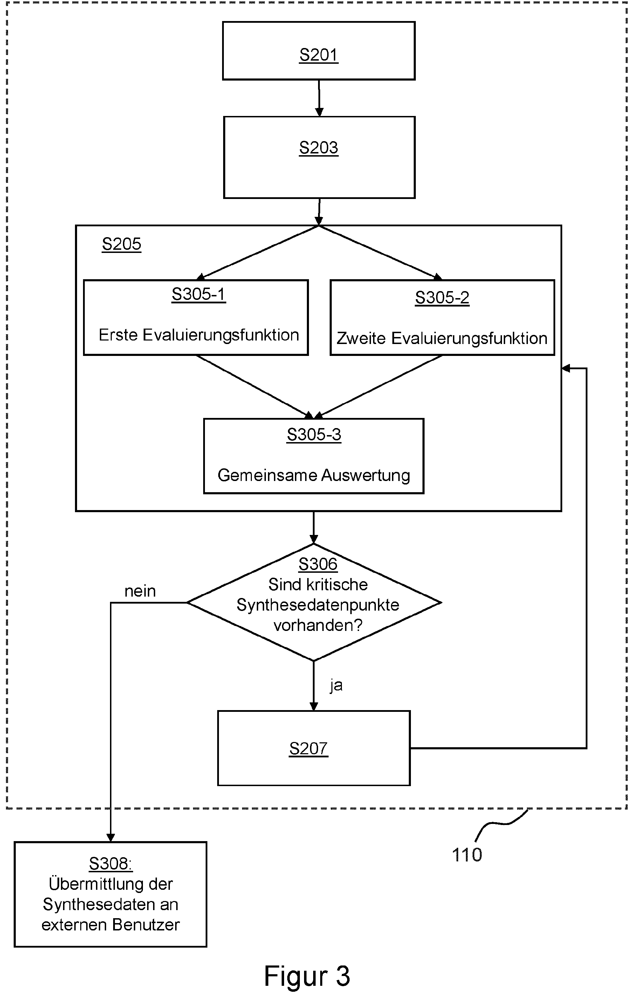

Ein Verfahren zur Bestimmung eines Grades an Privatheit und zur Veränderung der Synthesedaten 122-2 ist in dem Instruktionsspeicher 124 in Form von Programmcode oder ähnlichem gespeichert und kann auf dem Prozessor ausgeführt werden. Bei Ausführung dieses im Instruktionsspeicher 124 gespeicherten Programmcodes wird das Verfahren zur Veränderung des Synthesedatensatzes ausgeführt, welches in Figur 2 dargestellt ist. Zusätzlich zeigt auch die im Weiteren beschriebene Figur 3 eine spezielle Ausführungsform des beschriebenen Verfahrens, ergänzt um einige optionale Merkmale und Verfahrensschritte, wie weiter unten in diesem Text beschrieben wird.

Figur 2 zeigt ein Ablaufdiagramm eines Verfahrens zur Veränderung eines Synthesedatensatzes. Das Verfahren aus Figur 2 kann beispielsweise durch die Datenverarbeitungsvorrichtung 120 aus Figur 1 implementiert werden.

Ein Verfahrensschritt umfasst hierbei das Erhalten S201 eines Originaldatensatzes 122-1. Der Originaldatensatz umfasst beispielsweise in tabellarischer Form eine Vielzahl, von Originaldatenpunkten, wobei jeder der Originaldatenpunkte, dargestellt als Zeilen der Tabelle, Originalwerte für eine Vielzahl von Attributen, dargestellt als Spalten der Tabelle, umfasst. Die Datenpunkte sind beispielsweise als Vektor dargestellt und die einzelnen Attribute oder Einträge der Datenpunkte können über den Vektor indiziert werden.

Ein weiterer Verfahrensschritt umfasst das Erhalten S203 eines basierend auf dem Originaldatensatz generierten Synthesedatensatzes. Der Synthesedatensatz wurde basierend auf dem Originaldatensatz erzeugt mit dem Ziel, dass der Synthesedatensatz statistische Eigenschaften des Originaldatensatzes möglichst genau abbildet, aber keine privatsphärenverletzenden bzw. sicherheitskritischen Attributkombinationen offenbart. Der Synthesedatensatz umfasst eine Vielzahl von Synthesedatenpunkten, ebenfalls in tabellarischer Form dargestellt oder darstellbar, wobei jeder der Synthesedatenpunkte Synthesewerte für eine Vielzahl der Attribute umfasst.

Wenn der Synthesedatensatz vor Beginn des Verfahrens bereits vorliegt, können die Schritte S201 und S203 auch in umgekehrter Reihenfolge oder gleichzeitig ausgeführt werden.

Basierend auf dem Originaldatensatz und dem Synthesedatensatz können dann in Schritt S205 kritische Synthesedatenpunkte bestimmt werden. Hierzu wird aus der Menge der Synthesedatenpunkte mindestens ein kritischer Synthesedatenpunkt durch eine gemeinsame Auswertung S305-3 einer ersten Evaluierungsfunktion S305-1 und einer zweiten Evaluierungsfunktion S305-2 des Originaldatensatzes und Synthesedatensatzes bestimmt S205.

Details der ersten Evaluierungsfunktion S305-1, der zweiten Evaluierungsfunktion S305-2 und der gemeinsamen Auswertung S305-3 der beiden Evaluierungsfunktionen sind im Weiteren mit Bezug auf Figur 3 beschrieben.

Basierend auf dem Ergebnis vom Schritt S205 erfolgt dann nachfolgend in einem weiteren Verfahrensschritt ein Verändern S207 von mindestens einem der kritischen Synthesedatenpunkte.

Das Verändern eines kritischen Synthesedatenpunkts kann beispielsweise durch Löschen des kritischen Synthesedatenpunkts erfolgen. Dies kann insbesondere vorteilhaft sein, wenn ein einzelner Synthesedatenpunkt einen Ausreißer des Originaldatensatzes reproduziert hat. D.h. der Abstand dieses kritischen Synthesedatenpunkts zu dem Ausreißer des Originaldatensatzes ist sehr klein, während die Abstände dieses kritischen Synthesedatenpunkts zu den nächsten Originaldatenpunkten deutlich größer sind.

Bei kritischen Synthesedatenpunkten, die sich innerhalb eines Clusters befinden, kann es auch schon ausreichen, den kritischen Synthesedatenpunkt zu verändern, indem die Attribute des kritischen Synthesedatenpunkts leicht verrauscht werden oder indem einzelne reproduzierte Attribute verändert werden.

Figur 3 zeigt eine weitere Ausgestaltung des Verfahrens aus Figur 2 mit weiteren optionalen Verfahrensschritten und Funktionalitäten. Figur 3 spezifiziert zusätzlich zu den in Figur 2 gezeigten Schritten, weitere Verfahrensschritte und zeigt außerdem, welche der gezeigten und im Folgenden beschriebenen Verfahrensschritte innerhalb der sicheren Umgebung 110 ausgeführt werden.

Ebenso wie bei der in Figur 2 gezeigten Ausführungsform werden zunächst in den Schritten S201 und S203 der Originaldatensatz und der Synthesedatensatz erhalten. Diese beiden Verfahrensschritte werden innerhalb der sicheren Umgebung 110 durchgeführt. Ein Maß für die Privatheit des Synthesedatensatzes kann dann im automatisierten Vergleich mit dem Originaldatensatz erfolgen. Außerdem können, basierend auf diesem automatisierten Vergleich von Original- und Synthesedatensatz, Synthesedatenpunkte identifiziert werden, die Datenlecks und Sicherheitsrisiken darstellen und diese Synthesedatenpunkte, die als kritische Synthesedatenpunkte bezeichnet werden, können dann wiederum verändert werden, um die Privatheit des Synthesedatensatzes 122-2 zu erhöhen.

Ziel des Verfahrens aus Figur 3 ist es, einen Synthesedatensatz 122-2, der frei von kritischen Synthesedatenpunkten ist, zu erhalten und diesen Synthesedatensatz 122-2, der konform mit den Anforderungen an die Privatheit bzw. Datensicherheit ist, an einen externen Benutzer oder eine externe Datenverarbeitungsvorrichtung 150 außerhalb der sicheren Umgebung 110 zu übermitteln.

Als Unterschritte des bereits im Zusammenhang mit Figur 2 beschriebenen Verfahrensschrittes des Bestimmens S205 der kritischen Synthesedatenpunkte, werden in Figur 3 die Verfahrensschritte die erste Evaluierungsfunktion S305-1, die zweite Evaluierungsfunktion S305-2 und die gemeinsamen Auswertung S305-3 der beiden Evaluierungsfunktionen betrachtet und nachfolgend beschrieben.

Die erste Evaluierungsfunktion S305-1 bestimmt für jeden der Synthesedatenpunkte aus dem Synthesedatensatz ein Distanz-Dichte-Verhältnis, welches bezüglich einer Abstandsmetrik das Verhältnis zwischen, einerseits, einem Abstand des jeweiligen Synthesedatenpunkts zum nächsten Originaldatenpunkt, und, andererseits, der Dichte der benachbarten Originaldatenpunkte des jeweiligen Synthesedatenpunkts.

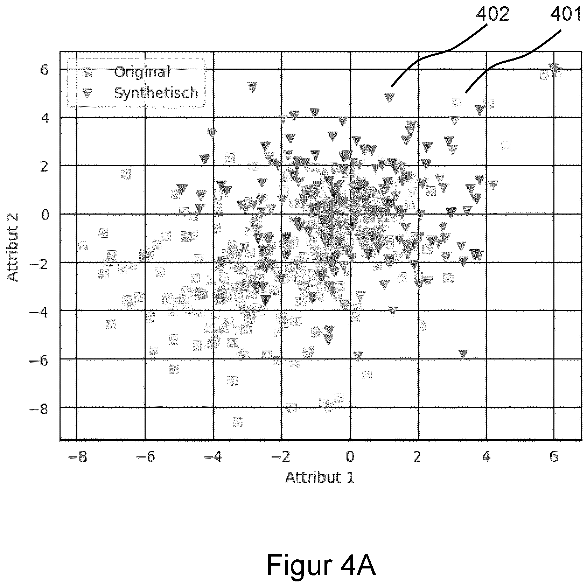

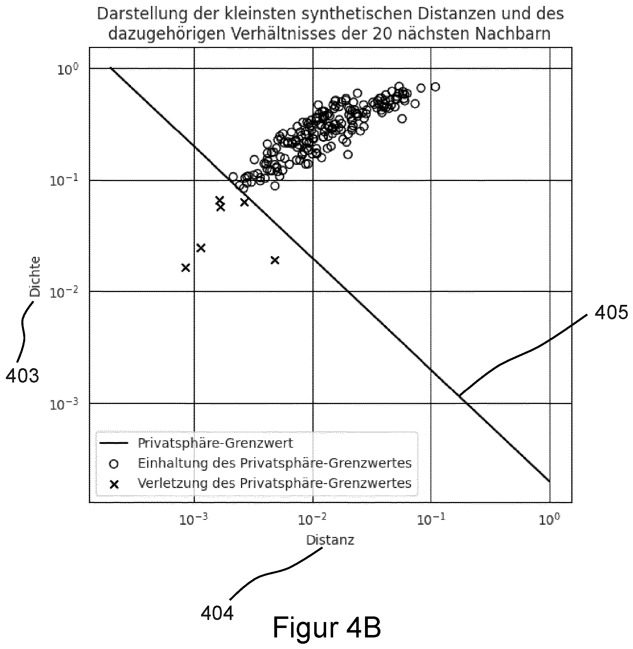

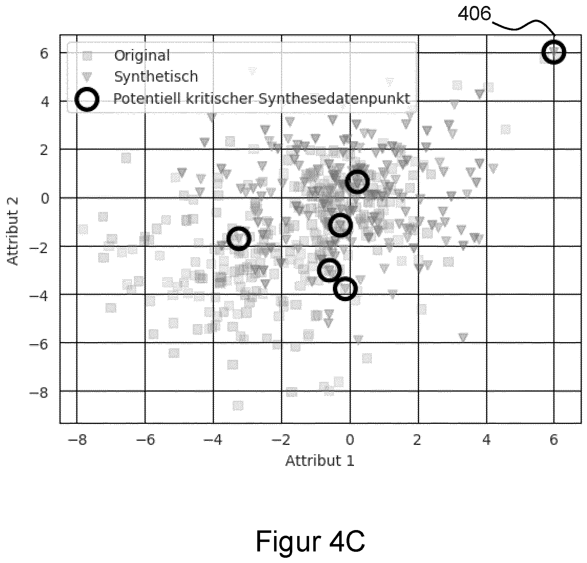

Als Abstandsmetrik kann hierbei vorzugsweise die oben bereits beschriebene Gower-Distanz verwendet werden. Details zum Distanz-Dichte-Verhältnis sind nachfolgend im Bezug zu Figuren 4A, 4B und 4C beschrieben.

Die zweite Evaluierungsfunktion S305-2 betrachtet Randverteilungen unwahrscheinlicher oder sogar einzigartiger Attributkombinationen. Hierzu werden zunächst einzigartige oder seltene Attributkombinationen im Originaldatensatz identifiziert. Hierzu können Attributkombination bestehend aus allen Attributen betrachtet werden sowie auch Kombinationen von Teilmengen aller Attribute oder auch einzelne Attribute. Bezüglich der seltenen Attributkombinationen des Originaldatensatzes wird dann überprüft, ob diese Attributkombinationen im Synthesedatensatz überrepräsentiert sind, also häufiger vorkommen als es statistisch der Fall sein sollte. Dazu werden die Randverteilungen der jeweiligen Attributkombination im Synthesedatensatz mit einem zweiten Schwellwert verglichen. Weitere Details dazu sind weiter unten in dieser Schutzrechtsanmeldung beschrieben.

Die erste Evaluierungsfunktion S305-1 und die zweite Evaluierungsfunktion S305-2 werden dann durch einen gemeinsame Auswertung S305-3 ausgewertet, um kritische Synthesedatenpunkte zu bestimmen. Hierbei kann die gemeinsame Auswertung S305-3 beispielsweise durch eine mehrschrittige Auswertung erfolgen, bei der erst die erste Evaluierungsfunktion S305-1 ausgewertet wird und dann auffällige Synthesedatenpunkte zusätzlich mit der zweiten Evaluierungsfunktion S305-2 ausgewertet werden (oder umgekehrt), oder es können beide Evaluierungsfunktionen unabhängig voneinander betrachtet werden und die jeweiligen auffälligen Synthesedatenpunkte zu den kritischen Synthesedatenpunkten zusammengefasst werden.

Nach der gemeinsamen Auswertung S305-3 wird in dem Verfahren entschieden S306, ob kritische Synthesedatenpunkte vorhanden sind. Vorhandene kritische Synthesedatenpunkte werden dann modifiziert S207 und das Verfahren zur erneuten Bestimmung kritischer Synthesedatenpunkte S205 kann wiederholt werden. Falls in dem Entscheidungsschritt S306 keine kritischen Synthesedatenpunkte vorhanden sind, so kann das Verfahren beendet werden und die Synthesedaten können an externe Benutzer übermittelt werden S308. Alternativ kann das Verfahren auch nach einer bestimmten Anzahl an Wiederholungen des Schritte S205 beendet werden.

Im Folgenden sind weitere Details zu den beiden Evaluierungsfunktionen beschrieben.

Figur 4A zeigt eine grafische Darstellung von einem Originaldatensatz 401 und Synthesedatensatz 402 mit jeweils drei Attributen. Die Originaldatenpunkte des Originaldatensatzes 401 sind als Vierecke dargestellt, die Synthesedatenpunkte des Synthesedatensatzes 402 als Dreiecke. Beide Datensätze verfügen über jeweils zwei numerische Attribute, die entlang der x-Achse und y-Achse dargestellt sind, sowie über ein kategorielles Attribut, welches anhand der Schattierung des jeweiligen Datenpunkts visualisiert ist.

Figur 4B zeigt eine Darstellung einer Implementierung der oben beschriebenen ersten Evaluierungsfunktion in Form eines Distanz-Dichte-Verhältnisses. Hierbei ist auf der x-Achse die Distanz 404 des jeweiligen Synthesedatenpunkts zu dem ihm am nächsten liegenden Originaldatenpunkt aufgetragen. Als Distanzmetrik wurde hierbei die Gower-Distanz verwendet. Auf der y-Achse ist als Maß für die Dichte 403 die mittlere Entfernung des jeweiligen Synthesedatenpunkts von den nächsten 20 Nachbarn aus dem Originaldatensatz, ohne den zuvor bereits verwendeten nächsten Originaldatenpunkt, aufgetragen.

Als kritische Synthesedatenpunkte werden in diesem Fall die Synthesedatenpunkte unterhalb des Schwellwerts, der in diesem Beispiel als Grenzwert-Linie 405 dargestellt ist, identifiziert. Bei diesen Synthesedatenpunkten ist die Entfernung zum nächsten Originaldatenpunkt im Verhältnis zur Dichte der umliegenden Originaldatenpunkte besonders klein, was auf eine Replikation eines Originaldatenpunkts im Synthesedatensatz hinweisen kann.

Der Schwellwert, bzw. die Grenzwert-Linie 405 kann entweder vor Beginn der Evaluierung gesetzt werden, oder sie kann anhand der real bestimmten Distanz-Dichte-Punktewolke so bestimmt werden, dass Punkte außerhalb eines Clusters als kritische Synthesedatenpunkte bestimmt werden.

In Figur 4C werden die sechs Synthesedatenpunkte, die in Figur 4B anhand der ersten Evaluierungsfunktion als kritische Synthesedatenpunkte 406 identifiziert wurden, markiert gezeigt. Hierbei ist zu sehen, dass manche der kritischen Synthesedatenpunkte in einem Bereich niedriger Dichte liegen, wie beispielsweise der markierte kritische Synthesedatenpunkt 406 oben rechts in der Grafik in Figur 4C. Auch die im Bild am tiefsten gelegenen markierten kritische Synthesedatenpunkte liegt in einem Bereich eher geringer oder mittlerer Dichte in Figure 4C. Die anderen markierten kritischen Synthesedatenpunkte liegen jedoch in Bereichen hoher oder sehr hoher Dichte. Da in Figur 4B das Distanz-Dichte-Verhältnis betrachtet wurde, muss aber bei den kritischen Synthesedatenpunkten, die in Bereichen hoher Dichte liegen, eine besonders große Ähnlichkeit mit dem ähnlichsten Originaldatenpunkt vorliegen. Üblicherweise wird eine vollständige oder fast vollständige Reproduktion eines Originaldatenpunkts auch in einem Bereich hoher Originalpunktdichte als nicht zulässig erachtet.

Um die Probleme der in Figur 4C gezeigten kritischen Synthesedatenpunkte zu beheben, könnten die kritischen Synthesedatenpunkte aus dem Datensatz entfernt werden oder es könnten einzelne Attribute leicht verändert werden. Alternativ oder zusätzlich könnten auch noch nachfolgend die Randverteilungen der zweiten Evaluierungsfunktion, für die anhand der ersten Evaluierungsfunktion bestimmten potenziell kritischen Synthesedatenpunkte, bestimmt werden. Ob die Synthesedatenpunkte verändert werden müssen, könnte dann durch eine gemeinsame Auswertung der Ergebnisse der ersten und zweiten Evaluierungsfunktion bestimmt werden.

Ein Datensatz definiert im statistischen Sinne eine gemeinsame Wahrscheinlichkeitsverteilung über allen Attributen, wobei somit im statistischen Sinne die Attribute als Zufallsvariablen der Wahrscheinlichkeitsverteilung betrachtet werden. Betrachtet man die Wahrscheinlichkeitsverteilungen einer Teilmenge der Attribute unter Fixierung einzelner Attribute, so erhält man Randverteilungen, bezüglich der fixierten Attribute. Bei nicht-kategoriellen und beispielsweise stetigen, numerischen Attributen können die Werte dieser stetigen Attribute in Klassen eingeteilt werden, um auch für stetige Attribute Randverteilungen betrachten zu können.

Die zweite Evaluierungsfunktion S305-2 betrachtet Randverteilungen unwahrscheinlicher oder sogar einzigartiger Attributkombinationen. Hierzu werden zunächst einzigartige oder seltene Attributkombinationen im Originaldatensatz identifiziert. Hierzu können Attributkombination bestehend aus allen Attributen betrachtet werden sowie auch Kombinationen von Teilmengen aller Attribute. Besonders kritisch sind hierbei einzigartige oder seltene Attributkombinationen. Wenn beispielsweise in einem Datensatz von 1.000 Patienten einer bestimmten Erkrankung nur 3 männliche Patienten enthalten sind, so ist der Wert "männlich" für das Attribut "Geschlecht" allein genommen eine seltene Attributkombination und die Synthesedatenpunkte mit dem Geschlecht "männlich" müssen entsprechend auf Datenlecks untersucht werden, da das Risiko besteht, dass die "männlichen" Originaldatenpunkte aufgrund ihrer Seltenheit von dem Synthesesystem reproduziert wurden und somit Rückschlüsse auf die Originaldaten zulassen könnten.

Bei numerischen Attributwerten ist es weiterhin möglich, die Attributwerteverteilung zu diskretisieren, indem Intervalle zusammengefasst werden. Hierbei können zusätzlich noch überlappende Intervalle betrachtet werden, wie in der statistischen Analyse üblich. Somit wird dann nicht jeder numerische Wert, der nur einmal vorkommt, als einzigartiges Attribut erkannt, sondern es werden vielmehr numerische Intervalle, in denen nur wenige Datenpunkte liegen, als seltene Attributwerte erkannt.

Die Betrachtung der Randverteilungen kann ebenfalls auf eine Datenschutzverletzung hinweisen und somit aufzeigen, ob ein Datensatz privatsphärewahrend synthetisiert worden ist oder nicht. Vor allem bei komplexen Daten sind einzigartige Attributkombinationen, welche Personen im Datensatz eindeutig identifizieren, möglich. Eine Möglichkeit zur Identifizierung einer solchen potenziellen Datenschutzverletzung ist die Untersuchung von bedingten empirischen Häufigkeiten für bestimmte Attributkombinationen. Diese Methode ermöglicht die Rekonstruktion und den Vergleich der bedingten Wahrscheinlichkeitsverteilung der gewählten Attributkombinationen des originalen und synthetischen Datensatzes. Hierbei sollten Kombinationen mit einer sehr geringen empirischen Häufigkeit im originalen Datensatz nicht reproduziert werden, da diese ggf. direkt auf Echtdaten zurückzuführen sind.

Hierbei ist insbesondere zu beachten, dass einzigartige Ausprägungen einer Attributkombination (oder auch Kombinationen mit einer sehr geringen Kardinalität) eines Originaldatensatz in den Synthesedaten niemals überrepräsentiert sein sollten, da dies auf ein Datenleck hindeuten kann.

Die Wahrscheinlichkeit, die seltenen Attributkombinationen in den synthetischen Daten zu beobachten, sollte somit zwischen 0 und $\frac{1}{n} + p$ , wobei $n$ die Anzahl an Dateneinträgen im Originaldatensatz und p die Wahrscheinlichkeit der zufälligen Erzeugung der spezifischen Attributkombination ist, liegen.

Somit ist vorzugsweise $p \ll \frac{1}{n'}$, um eine Anwendung der statistischen Tests der zweiten Evaluierungsfunktion zu ermöglichen.

Wenn es beispielsweise k boolesche Attributwerte gibt, die aus den Daten entnommen wurden, und alle möglichen Kombinationen unabhängig voneinander entnommen wurden (was für große k vernünftig erscheint, da die Zahl der möglichen Attributkombinationen exponentiell ansteigt, so dass ein Synthesemodell nicht alle Kombinationen in voreingenommener Weise entnehmen kann), ist die Wahrscheinlichkeit, eine bestimmte Kombination zu beobachten, im schlimmsten Fall gleich $2^{-k}$.

Solange also $2^k \gg n$ ist, kann das Durchsickern von persönlichen Daten in dem Synthesedatensatz mit der zweiten Evaluierungsfunktion auf Basis der Randverteilungen erkannt werden.

Es kann dann ein einfacher Chi-Quadrat-Test oder ein Binomialtest durchgeführt werden, um zu prüfen, ob sich die Verteilungswahrscheinlichkeit der Attributkombinationen signifikant von 0 unterscheidet. Dieser Test kann für möglichst viele seltene Attributkombinationen wiederholt werden.

Die beiden in der ersten und zweiten Evaluierungsfunktion abgebildeten Verfahren zur Detektion von Datenlecks können für belastbare Analysen der Privatsphäre in Kombination angewandt werden. Hierfür gibt es mehrere exemplarische Szenarien:

1. Beide Evaluierungsfunktionen zeigen keine Auffälligkeiten, d.h. es werden keine kritischen Synthesedatenpunkte identifiziert. In diesem Fall kann der Synthesedatensatz privatsphärewahrend und/oder ohne Sicherheitsprobleme an externe Benutzer außerhalb der sicheren Umgebung übertragen werden.

2. Das Distanz-Dichte-Verhältnis, als Ausgestaltung der ersten Evaluierungsfunktion, zeigt Auffälligkeiten. In diesem Fall können die Attributkombinationen der mittels des Distanz-Dichte-Verhältnisses identifizierten Synthesedatenpunkte mit der zweiten Evaluierungsfunktion auf Auffälligkeiten bei den Randverteilungen getestet werden. Falls die mittels Distanz-Dichte-Verhältnis identifizierten Synthesedatenpunkte auch bzgl. Ihrer Attributkombinationen auffällige, d.h. unwahrscheinliche, Randverteilungen zeigen, so werden diese Synthesedatenpunkte als kritische Synthesedatenpunkte bestimmt und verändert oder entfernt.

3. Die Randverteilungsmetrik, als Ausgestaltung der zweiten Evaluierungsfunktion, weist Auffälligkeiten auf. In diesem Fall wird das Distanz-Dichte-Verhältnis spezifisch für die auffälligen Attributkombinationen erneut berechnet. Falls dieses spezielle Distanz-Dichte-Verhältnis ebenfalls auffällige Synthesedatenpunkte zeigt, d.h. Synthesedatenpunkte, deren Distanz-Dichte-Verhältnis der auffälligen Attributkombinationen unterhalb eines Schwellwertes liegt, so werden diese Synthesedatenpunkte als kritische Synthesedatenpunkte bestimmt und entweder verändert oder aus dem Synthesedatensatz entfernt.

4. Beide Evaluierungsfunktionen weisen bezüglich bestimmter Synthesedatenpunkte Auffälligkeiten auf. In diesem Fall ist es vorteilhaft diese kritischen Synthesedatenpunkte zu löschen oder eine erneute Generierung des Synthesedatensatzes mit veränderten Parametern durchzuführen.

Durch die Kombination beider Metriken, die durch die erste und zweite Evaluierungsfunktionen umgesetzt werden, ist es somit möglich, die gesamte Privatsphäre des Synthesedatensatzes zu evaluieren und eine selektive Veränderung des Synthesedatensatzes zu ermöglichen, wodurch Datenlecks vermieden und Synthesedaten, anstatt der Originaldaten, veröffentlicht werden können.

Bezugzeichenliste

| | |
|---|---|
| 100 | System |
| 110 | sichere Umgebung |
| 120 | Datenverarbeitungsvorrichtung |
| 122 | Datenspeicher von 120 |
| 122-1 | Originaldatensatz |
| 122-2 | Synthesedatensatz |
| 124 | Instruktionsspeicher von 120 |
| 126 | Prozessor von 120 |
| 128 | Kommunikationsschnittstelle von 120 |
| 130 | sichere externe Datenverarbeitungsvorrichtung |

| | |
|---|---|
| 132 | Datenspeicher von 130 |
| 134 | Instruktionsspeicher von 130 |
| 136 | Prozessor von 130 |
| 138 | Kommunikationsschnittstelle von 130 |
| 140 | externe Geräte |
| 150 | externe Datenverarbeitungsvorrichtung |
| 152 | Datenspeicher von 150 |
| 154 | Instruktionsspeicher von 150 |
| 156 | Prozessor von 150 |
| 158 | Kommunikationsschnittstelle von 150 |
| S201 | Erhalten des Originaldatensatzes |
| S203 | Erhalten des Synthesedatensatze |
| S205 | Bestimmen der kritischen Synthesedatenpunkte |
| S207 | Verändern der kritischen Synthesedatenpunkte |
| S305-1 | erste Evaluierungsfunktion |
| S305-2 | zweite Evaluierungsfunktion |
| S305-3 | gemeinsame Auswertung |
| S306 | Überprüfung auf kritische Synthesedatenpunkte |
| S308 | Übermittlung Synthesedatensatz |
| 401 | Originaldatenpunkt |
| 402 | Synthesedatenpunkt |
| 403 | Dichte |
| 404 | Distanz |
| 405 | Schwellwert |
| 406 | kritischer Synthesedatenpunkt |

**Patentansprüche**

1.  Computerimplementiertes Verfahren zur selektiven Veränderung eines Synthesedatensatzes, das Verfahren umfassend die folgenden Schritte:

    a) Erhalten (S201) eines Originaldatensatzes, umfassend eine Vielzahl von Originaldatenpunkten, wobei jeder der Originaldatenpunkte Originalwerte für eine Vielzahl von Attributen umfasst;
    b) Erhalten (S203) eines basierend auf dem Originaldatensatz generierten Synthesedatensatzes, umfassend eine Vielzahl von Synthesedatenpunkten, wobei jeder der Synthesedatenpunkte Synthesewerte für eine Vielzahl der Attribute umfasst;
    c) Bestimmen (S205) einer Menge kritischer Synthesedatenpunkte aus der Vielzahl der Synthesedatenpunkte durch eine gemeinsame Auswertung (S305-3) einer ersten Evaluierungsfunktion (S305-1) und einer zweiten Evaluierungsfunktion (S305-2) des Originaldatensatzes und Synthesedatensatzes,

    - Wobei die erste Evaluierungsfunktion (S305-1) bezüglich einer ersten Abstandsmetrik ein Verhältnis von einem Abstand zwischen jeweils einem Synthesedatenpunkts und mindestens einem benachbarten Originaldatenpunkt zu einer Dichte benachbarter Originaldatenpunkte dieses Synthesedatenpunkts mit einem ersten Schwellwert vergleicht;
    - Wobei die zweite Evaluierungsfunktion (S305-2) für mindestens eine seltene Attributkombination des Originaldatensatzes eine Randverteilung dieser seltenen Attributkombination des Synthesedatensatzes bestimmt und mit mindestens einem zweiten Schwellwert vergleicht; und

    d) Verändern (S207) mindestens eines der kritischen Synthesedatenpunkte.

2.  Verfahren gemäß Anspruch 1, weiterhin umfassend, nach dem Verändern von dem mindestens einen der kritischen Synthesedatenpunkte,

    e) Wiederholen von Schritt c) basierend auf dem Originaldatensatz und einem veränderten Synthesedatensatz, der die veränderten kritischen Synthesedatenpunkte umfasst;
    f) wenn erneut kritische Synthesedatenpunkte bestimmt werden: Wiederholen von Schritt d);
    g) ansonsten: Beenden des Verfahrens.

3. Computerimplementiertes Verfahren gemäß einem der vorherigen Ansprüche, wobei das Verändern eines kritischen Synthesedatenpunkts umfasst: Löschen des kritischen Synthesedatenpunkts aus dem Synthesedatensatz, Verändern des Synthesewerts mindestens eines Attributs des Synthesedatenpunkts, Verändern der Synthesewerte aller Attribute des Synthesedatenpunkts.

4. Computerimplementiertes Verfahren gemäß einem der vorherigen Ansprüche, wobei die Attribute der Originaldatenpunkte zumindest eine Teilmenge folgender Datenarten umfassen: persönliche Daten wie Größe, Gewicht, ethnische Zugehörigkeit, Adressdaten, Bilddaten, GPS-Daten, Bewegungsprofile, medizinische Daten und/oder Blutwerte.

5. Computerimplementiertes Verfahren gemäß einem der vorherigen Ansprüche, wobei ein Synthesedatenpunkt als kritischer Synthesedatenpunkt bestimmt wird, wenn die erste Evaluierungsfunktion bezüglich dieses Synthesedatenpunkts oberhalb des ersten Schwellwert und/oder die zweite Evaluierungsfunktion bezüglich dieses Synthesedatenpunkts oberhalb des zweiten Schwellwerts liegt.

6. Computerimplementiertes Verfahren gemäß einem der vorherigen Ansprüche: wobei das Bestimmen, dass ein Synthesedatenpunkt ein kritischer Synthesedatenpunkt ist, weiterhin beinhaltet:

   - Bestimmen, dass die erste Evaluierungsfunktion bezüglich dieses Synthesedatenpunkts oberhalb des ersten Schwellwerts liegt und, nachfolgend,
   - Bestimmen von Randverteilungen von Attributkombinationen dieses Synthesedatenpunkts und Vergleichen der Randverteilungen mit Schwellwerten;
   und/oder
   - Bestimmen, dass die zweite Evaluierungsfunktion bezüglich einer Attributkombination der Synthesedatenpunkte oberhalb des zweiten Schwellwerts liegt und, nachfolgend,
   - Bestimmen von der ersten Evaluierungsfunktion basierend auf einem auf die auffällige Attributkombination eingeschränkten Originaldatensatz und Synthesedatensatz.

7. Computerimplementiertes Verfahren gemäß einem der vorherigen Ansprüche, weiterhin umfassend die folgenden Verfahrensschritte:

   - Bestimmen des ersten Schwellwerts basierend auf dem Originaldatensatz und/oder Synthesedatensatz; und/oder
   - Bestimmen des zweiten Schwellwerts basierend auf dem Originaldatensatz und/oder Synthesedatensatz.

8. Computerimplementiertes Verfahren gemäß einem der vorherigen Ansprüche, weiterhin umfassend, vor dem Erhalten des Synthesedatensatzes,

   - Generieren des Synthesedatensatzes basierend auf dem Originaldatensatz.

9. Computerimplementiertes Verfahren gemäß Anspruch 8, wobei der Synthesedatensatz mittels eines generativen ML-Models, das vorab basierend auf den Originaldaten trainiert wurde, generiert wird.

10. Computerimplementiertes Verfahren gemäß einem der vorherigen Ansprüche, weiterhin umfassend: wenn keine kritischen Synthesedatenpunkte bestimmt werden, Übermittlung der Synthesedaten an einen externen Benutzer.

11. Datenverarbeitungsvorrichtung (120) zur selektiven Veränderung eines Synthesedatensatzes (122-2), die Datenverarbeitungsvorrichtung (120) umfassend einen Prozessor (126), einen Datenspeicher (122) und einen Instruktionsspeicher (124),

   der Datenspeicher (122) konfiguriert zum Speichern eines Originaldatensatzes (122-1) und eines, basierend auf dem Originaldatensatz (122-1) generierten, Synthesedatensatzes (122-2), der Originaldatensatz (122-1), umfassend eine Vielzahl von Originaldatenpunkten, wobei jeder der Originaldatenpunkte Originalwerte für eine Vielzahl von Attributen umfasst; der Synthesedatensatz (122-2), umfassend eine Vielzahl von Synthesedatenpunkten, wobei jeder der Synthesedatenpunkte Synthesewerte für eine Vielzahl der Attribute umfasst; der Instruktionsspeicher (124) konfiguriert zum Speichern von Programminstruktionen, die wenn sie auf dem Prozessor (126) der Datenverarbeitungsvorrichtung (120) ausgeführt werden, die Datenverarbeitungsvorrichtung (120) veranlassen, das folgende Verfahren auszuführen:

- Bestimmen (S205) einer Menge kritischer Synthesedatenpunkte durch eine gemeinsame Auswertung einer ersten Evaluierungsfunktion und einer zweiten Evaluierungsfunktion,
- Verändern (S207) mindestens eines der kritischen Synthesedatenpunkte;
- Wobei die erste Evaluierungsfunktion bezüglich einer ersten Abstandsmetrik ein Verhältnis von einem Abstand zwischen jeweils einem Synthesedatenpunkts und mindestens einem benachbarten Originaldatenpunkt zu einer Dichte benachbarter Originaldatenpunkte dieses Synthesedatenpunkts mit einem ersten Schwellwert vergleicht;
- Wobei die zweite Evaluierungsfunktion für mindestens eine seltene Attributkombination des Originaldatensatzes eine Randverteilung dieser seltenen Attributkombination des Synthesedatensatzes bestimmt und mit mindestens einem zweiten Schwellwert vergleicht.

12. Computersystem zur Distribution eines Synthesedatensatzes umfassend eine erste Datenverarbeitungsvorrichtung zur selektiven Veränderung eines Synthesedatensatzes gemäß Anspruch 9 und weiterhin umfassend eine zweite Datenverarbeitungsvorrichtung, wobei die erste Datenverarbeitungsvorrichtung in einer sicheren Umgebung angeordnet ist und die zweite Datenverarbeitungsvorrichtung außerhalb der sicheren Umgebung angeordnet ist, wobei die erste Datenverarbeitungsvorrichtung weiterhin konfiguriert ist, den veränderten Synthesedatensatz an die zweite Datenverarbeitungsvorrichtung zu übertragen.

13. Computersystem gemäß Anspruch 13, weiterhin umfassend, nach dem Verändern von dem mindestens einem der kritischen Synthesedatenpunkte und vor dem Übertragen des veränderten Synthesedatensatzes an die zweite Datenverarbeitungsvorrichtung (150):

- Wiederholen des Bestimmens (S205) einer Menge kritischer Synthesedatenpunkte durch eine gemeinsame Auswertung einer ersten Evaluierungsfunktion und einer zweiten Evaluierungsfunktion,
- wenn erneut kritische Synthesedatenpunkte bestimmt werden: Wiederholen des Veränderns mindestens eines der kritischen Synthesedatenpunkte.

14. Computerprogrammprodukt, umfassend Befehle, die bei der Ausführung des Programms durch einen Computer diesen veranlassen, die Schritte des Verfahrens gemäß Anspruch 1 bis 9 auszuführen.

Figur 1

Erhalten eines Originaldatensatzes, umfassend eine Vielzahl von Originaldatenpunkten, wobei jeder der Originaldatenpunkte Originalwerte für eine Vielzahl von Attributen umfasst;  ⌇ S201

Erhalten eines basierend auf dem Originaldatensatz generierten Synthesedatensatzes, umfassend eine Vielzahl von Synthesedatenpunkten, wobei jeder der Synthesedatenpunkte Synthesewerte für eine Vielzahl der Attribute umfasst;  ⌇ S203

Bestimmen einer Menge kritischer Synthesedatenpunkte aus der Vielzahl der Synthesedatenpunkte durch eine gemeinsame Auswertung einer ersten Evaluierungsfunktion und einer zweiten Evaluierungsfunktion des Originaldatensatzes und Synthesedatensatzes,  ⌇ S205

Verändern mindestens eines der kritischen Synthesedatenpunkte.  ⌇ S207

# Figur 2

Figur 3

Figur 4A

Darstellung der kleinsten synthetischen Distanzen und des dazugehörigen Verhältnisses der 20 nächsten Nachbarn

403

405

404

Figur 4B

Figur 4C

**EUROPÄISCHER RECHERCHENBERICHT**

Europäisches Patentamt
European Patent Office
Office européen des brevets

Nummer der Anmeldung

EP 25 18 6551

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2021/326652 A1 (HAZARD CHRISTOPHER JAMES [US] ET AL) 21. Oktober 2021 (2021-10-21) * Absätze [0125] - [0232] * * Absätze [0082], [0199], [0324], [0328], [0349], [0359], [0376], [0384] * * Abbildungen 3,6 * ----- | 1-14 | INV. G06F18/28 G06F21/62 G06F17/18 |
| A | ZILONG ZHAO ET AL: "CTAB-GAN: Effective Table Data Synthesizing", ARXIV.ORG, CORNELL UNIVERSITY LIBRARY, 201 OLIN LIBRARY CORNELL UNIVERSITY ITHACA, NY 14853, 31. Mai 2021 (2021-05-31), XP081953691, * Zusammenfassung * * Abschnitt 4.2.3 * * Abbildungen 2,5,6 * ----- | 1,8,9, 11,14 | |

**RECHERCHIERTE SACHGEBIETE (IPC)**

G06F
G16H

Der vorliegende Recherchenbericht wurde für alle Patentansprüche erstellt

| Recherchenort | Abschlußdatum der Recherche | Prüfer |
|---|---|---|
| Den Haag | 4. September 2025 | Hermes, Lothar |

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 25 18 6551

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

04-09-2025

| Im Recherchenbericht angeführtes Patentdokument | Datum der Veröffentlichung | Mitglied(er) der Patentfamilie | Datum der Veröffentlichung |
|---|---|---|---|
| US 2021326652 A1 | 21-10-2021 | KEINE | |

EPO FORM P0461

Für nähere Einzelheiten zu diesem Anhang : siehe Amtsblatt des Europäischen Patentamts, Nr.12/82